(19) Europäisches Patentamt
European Patent Office
Office européen des brevets

(11) EP 2 035 459 B1

(12) **EUROPEAN PATENT SPECIFICATION**

(45) Date of publication and mention of the grant of the patent:
**16.12.2009 Bulletin 2009/51**

(21) Application number: **07734630.2**

(22) Date of filing: **22.05.2007**

(51) Int Cl.:
***C08B 15/00*** *(2006.01)* ***A61L 15/28*** *(2006.01)*
***D06M 13/325*** *(2006.01)*

(86) International application number:
**PCT/IB2007/001322**

(87) International publication number:
**WO 2007/135548 (29.11.2007 Gazette 2007/48)**

(54) **COVALENT CONJUGATES OF COTTON AND SUBSTITUTES (VISCOSE, MODAL COTTON) WITH BIOACTIVE SUBSTANCES HAVING ANTISEPTIC, SANITIZING, ACARICIDAL AND INSECT REPELLENT ACTIVITY, AND A METHOD FOR OBTAINING THEM**

KOVALENTE KONJUGATE VON BAUMWOLLE UND ERSATZSTOFFEN (VISKOSE, MODAL-BAUMWOLLE) MIT BIOAKTIVEN SUBSTANZEN MIT ANTISEPTISCHER, DESINFIZIERENDER, AKARIZIDER UND INSEKTENABWEHRENDER WIRKUNG UND HERSTELLUNGSVERFAHREN DAFÜR

CONJUGUÉS COVALENTS DE COTON ET DE SUBSTITUTS (VISCOSE, COTON MODAL) À SUBSTANCES BIOACTIVES AYANT UNE ACTIVITÉ ANTISEPTIQUE, DÉSINFECTANTE, ACARICIDE ET INSECTIFUGE, ET LEURS PROCÉDÉS D'OBTENTION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **23.05.2006 IT MI20061014**

(43) Date of publication of application:
**18.03.2009 Bulletin 2009/12**

(73) Proprietor: **Franzoni Filati S.p.A.**
**25122 Brescia (IT)**

(72) Inventors:
- **VOLPATO, Ivo**
  **I-06070 San Mariano (IT)**
- **BIZZINI, Bernard**
  **F-11000 Carcassonne (FR)**
- **FRANZONI, Maria Grazia**
  **I-25040 Cortefranca (IT)**

(74) Representative: **Gervasi, Gemma et al**
**Notarbartolo & Gervasi S.p.A.**
**Corso di Porta Vittoria 9**
**20122 Milano (IT)**

(56) References cited:
**EP-A- 1 424 087**
**WO-A-01/97617**
**US-A- 6 077 319**
**US-A1- 2003 056 297**
**US-A1- 2004 166 144**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The use of acaricides, insecticides/insect repellents and microbicides in common marketable forms (powders, liquids, sprays) can create intolerance problems particularly in allergic individuals.

On the other hand, mites, insects and microbes are actual living etiological causes of allergic pathologies.

### STATE OF THE ART

Living forms such as mites and insects, and microorganisms such as moulds and bacteria which are also responsible for infections, are included among the etiological causes of allergic pathologies.

In order to prevent allergies caused by these forms, recourse is made to substances such as acaricides, insecticides/insect repellents and antimicrobial/antifungal agents, which are generally applied within the environment as powders or sprays.

However, the use of these marketable forms is itself a risk factor; in this respect, the dispersal of bioactive molecules such as acaricides, insecticides/insect repellents and antiseptics/microbicides within the environment can be a further factor in triggering an allergic episode, particularly for atopic individuals.

An additional comment must be made regarding those antimicrobials (antiseptics, antifungals etc.) which also find application as disinfectants for wounds and sores, and hence have topical-mucosal use. In this case, they are applied in the form of a liquid, gel or ointment and the disinfected area is then covered with a bandage or gauze of cotton, non-woven fabric or similar materials.

Applying antiseptics in the form of a liquid or barrier cream has been observed to significantly delay the tissue repair process, as has also been observed that covering with bandages and gauzes, though sterile, is a risk factor for infection since woven and non-woven fabrics function as breeding-grounds for microbes. Marketable forms of gauzes and bandages, in which antiseptics/microbicides are adsorbed or variously entrapped within the stitches of the protective fabric, do not improve the situation in that said substances can be released and diluted in the serum substrate of wound repair with consequent reduction in effectiveness over time and onset of local immunosuppressive side effects which can delay the repair process itself.

Therefore, as is apparent from the aforestated, the need exists to provide a method for fixing bioactive substances such as microbicides, acaricides, insecticides or insect repellents which leads to a lasting and effective attachment under the conditions of use. Also, it is critical that the attachment allows the bioactive substance to substantially maintain its activity.

US 4035146 proposes to resolve these problems by the attachment of bioactive substances to non-derivatized supports bearing OH groups, through the linker 2,4,6-tri-chloro 1,3,5 triazine. Although not having to modify (or activate) the support prior to attachment is a mild strategy and hence desirable from the technical viewpoint, it is also true that, to the Applicant's knowledge, substrates modified with antimicrobial substances attached through 2,4,6-tri-chloro 1,3,5 triazine are not commercially available to date. Furthermore, this linker, with an LD 50 (rat) of 930 mg/kg is in the same classification as substances whose handling involves a health risk.

In a preceding invention, plant polymers such as cellulose and cotton, in their various marketable forms: fibre, yarn, non-woven or woven fabric, were considered as substrates for the conjugation of bioactive molecules with the aim of obtaining pharmaceutical, cosmetic or sanitary products or products for use in the food industry.

One aspect of said invention was to provide a method for conjugating bioactive molecules to the plant polymer in these various forms: fibre, yarn, woven or non-woven fabric, which comprised:

a. creation of sites suitable for chemical conjugation on the polysaccharide polymer, in its various marketable forms, to obtain an activated polysaccharide polymer;

b. reaction between the activated polysaccharide polymer and a bioactive substance or a derivative thereof to form a chemical conjugate;

c. optional modification of the nature of the chemical conjugation by a chemical reaction; this could happen by forming a direct chemical bond between the polymer and the bioactive molecule or by inserting at least bivalent groups, so called linkers, which allow the conjugation of a greater number of bioactive molecules to a site in the plant polymer.

The nature of the conjugation bonds could be different depending on the need, or otherwise, for the conjugated bioactive molecule to be released (bioavailability).

Preferred linkers were found to be lysine, aspartic acid, glutamic acid, thio-threonine type molecules or polylinkers.

One of the preferred methods of the invention comprised, for example:

a. partial oxidation of the primary alcohol -OH groups present in the polysaccharide polymer;
b. reaction between the activated polymer, comprising aldehyde groups, and lysine with formation of the Schiff base;
c. optional reduction of the bond;
d. conjugation of the amino group with a carbonyl group of the bioactive molecule. Preferred conjugatable bioactive substances were defined as being those characterized by the presence of an amino, carboxyl, thiol or carbonyl group.

In conclusion, the invention therefore concerned various conjugation schemes, the use of various types of linkers and various reaction conditions dependent on the bioactive molecule to be conjugated and the degree of bioavailability that it must possess.

In the specific case of acaricide and insecticide/insect repellent molecules used in particular for preventing allergic episodes, and antimicrobial molecules, used for the same purpose and also for disinfecting wounds, we have already stated that these do not have to be released, and hence need not be bioavailable, but must carry out their microbicidal or larvicidal activity directly at the conjugation site (fibre, woven fabric, garment, outdoor accessory, non-woven fabric for various uses); the optimal bond for achieving this result is the chemical covalent bond between plant polymer and bioactive molecule.

Since the bioactive substances, with acaricidal, microbicidal and insecticidal/insect repellent action, have substantially different structures, as the marketable properties of the various polymers of cotton and substitutes also differ, it could be assumed, based on the state of the art, that different conjugation procedures and conditions were needed to achieve irreversible binding of the bioactive molecule at suitable concentrations for optimal performance. In view of this situation it was instead desirable to provide a new industrial scale procedure, achievable using normal equipment already present in the textile industry used for classical treatments and pre-treatments of the supports herein discussed, such as hydrophilization, bleaching or dyeing. Ideally, said procedure should completely safeguard the organoleptic and marketable properties of the supports. Also desirable was the provision of new supports derivatized by means of the new procedure and characterized on the one hand by having better protection, under usage conditions (such as washing), against release of the bioactive substance bound to the support, and on the other hand by a considerable preservation of its activity.

## SUMMARY OF THE INVENTION

The inventors have now surprisingly found that, by using forced circulation dyeing reactors used for dyeing fibres and yarn, or shaking dye baths, used for dyeing textiles and finished garments, it is possible to standardize the conditions and the industrial process for covalently conjugating structurally different bioactive molecules having microbicidal, acaricidal, insecticidal/insect repellent action, to fibre, yarn, woven fabric, and finished articles of cotton and its substitutes.

In particular the new industrial scale process of covalently conjugating bioactive substances, chosen from the group consisting of microbicides, acaricides, insecticides and/or insect repellents which bear or are derivatized to bear an amino or halogen group, to supports of fibre, yarn, woven or non-woven fabric of cotton or substitutes identified by the inventors, comprises the following steps:

- pre-treatment of the support, suitable for creating aldehyde groups on its surface
- introduction of the bioactive substance bearing or derivatized to bear an amino group, so as to enable the pre-treated support and bioactive substance to react together;
- or, as an alternative to the preceding step, introduction of a linker that bears at least two amino groups, so as to enable the pre-treated support and linker to react together;
- reduction, by introducing a reducing agent, of the bond formed between the support and bioactive substance, or, in the alternative case, between the surface and linker, as well as of any excess aldehyde groups on the support.
- optionally in the case of said alternative, introducing a bioactive substance bearing or derivatized to bear a halogen group, so as to enable the linker previously bound to the support and the bioactive substance to react together.

The industrial scale process comprises a partial pre-oxidation of the support in cotton and substitutes in their various marketable forms, hydrophobic and hydrophilic fibre, hydrophobic and hydrophilic yarn, fabric and finished articles, which is conducted under specific conditions such that the organoleptic and marketable properties of the polymers are not changed. This requirement is of great importance, the reason being that in the past, approaches of the type described in US 4035146 were often favoured, especially for industrial scale processes. The conjugation of the various bioactive molecules, either directly in the form of an aminated derivative, or alternatively through linkers of diamine, triamine or tetramine structure, lysine, melamine, polymerized melamine or tetraaminobenzene, takes place, in a first

step, via Schiff base formation between the aldehyde group previously created on the surface of the support and an amino group of the linker (or active principle) used. Subsequently, the newly formed bonds, as well as any excess oxidized sites present on the substrate, are reduced. After which, if linkers are used, conjugation of the various bioactive molecules in their halogenated form takes place.

Conjugates obtained in this manner are extremely stable even after numerous washes under conditions of chemical and thermal stress, with the desired activity also being maintained.

As previously stated, the procedure is useful for all the various hydrophilic and hydrophobic marketable forms of the polymer: fibre, yarn, fabric, finished articles, and can be applied either before, simultaneous to or following the normal dyeing processes.

The process can be undertaken in normal dyeworks.

Surprisingly, the use of tri and tetramine linkers optimises at the same time their reactivity to dyes and improves the organoleptic characteristics of the finished dyed article. In particular, it has been surprisingly found that the use of melamine, melamine polymerized as described herein and tetraaminobenzene as linkers also act as coadjuvants in textile dyeing processes.

## DETAILED DESCRIPTION OF THE INVENTION

The Invention concerns the use of cotton and its substitutes, viscose and modal cotton, as a substrate for the use of bioactive molecules, of microbicidal, acaricidal, insecticidal and insect repellent type, without their becoming dispersed within the environment or released onto the skin, hence avoiding possible side effects connected to their use.

A distinctive feature of covalently fixing the suitably derivatized bioactive molecules described herein to substrates of cotton or substitutes used for the production of bandages, gauzes, garments, outdoor accessories, non-wovens and the like, is that it allows their effectiveness to be preserved while preventing their release into the environment and contact with skin, hence eliminating the risks of side effects.

The covalent conjugation procedure comprises a cotton or substitute pre-oxidation step; subsequent conjugation of the bioactive molecules which can either take place directly, by reacting their amino-derivatives, or through amino linkers, and subsequent reaction with their halogen derivatives; reduction of the bonds.

The function of diamine, triamine, tetramine or polyamine type linker bonds is to rationalize the active concentration of bioactive molecule conjugated to cotton in order to optimise its effectiveness.

The relative covalent bond obtained is stable even after numerous machine washes, enabling its effectiveness to be retained for long periods of time.

The covalent conjugation procedure is conducted in normal reactors used in yarn and fabric dyeing processes.

Said procedure is therefore compatible with yarn and fabric dyeing processes which can be carried out directly before or after covalent conjugation of the bioactive molecules.

In particular, the objectives discussed above were achieved by way of a specific procedure of covalently conjugating the bioactive molecules, suitably derivatized where necessary, to fibre, yarn or fabrics of cotton and substitutes, which comprises, as previously mentioned:

a. a partial pre-oxidation of the polymer suitable for preserving its organoleptic and marketable processability properties;
b. attachment, to the pre-oxidised polymer, of amino derivatives of the bioactive molecules or amino type linker bonds with bridging function selected to rationalize the concentration of bioactive molecule to be bound per unit weight of polymer;
c. reduction of those pre-oxidized groups having not reacted with the amino derivative of the bioactive molecule or with the linker, as well as reduction of the bonds formed between pre-oxidized groups and the linker;
d. final conjugation, where linkers are used, of the bioactive molecule in the form of a halogen derivative able to react with the free amino groups of the linkers.

The pre-oxidation is preferably conducted using sodium periodate (sodium meta-periodate) as oxidizing agent, at a concentration of between 0.02 M and 0.2 M, preferably 0.08 M for a period of between 5 and 30 minutes, preferably 15 minutes, for cotton and modal cotton, and a period of between 5 and 120 minutes, preferably 30 minutes for viscose.

The reaction is conducted at a temperature between ambient T °C and 70°C, preferably at 50°C, and at a pressure between atmospheric P and 5 bar, preferably 3.5 bar.

Depending on the bioactive molecules to be bound, the following are used as linker agents: diamines of lysine monohydrochloride type, tetramines of tetraaminobenzene type (preferably 1,2,3,5-tetraaminobenzene). Melamine can be used as such or in its polymerized form to constitute a polyamine. In this case a controlled polymerization of melamine is carried out with a dialdehyde, such as glutaraldehyde, measuring the dialdehyde sub-stoichiometrically to obtain copolymer chains bearing lateral amino groups, which remain thus available for the conjugation of molecules of halogenated bioactive substance. Preferably melamine copolymerized with dialdehyde, in particular glutaraldehyde, is used, in a sub-stoichiometric ratio and having a m.w. of between 2000 and 10,000 Da.

One of the amino groups on the linker (which always bears at least two of them) is used for bonding to the aldehyde group of the pre-oxidized cotton or substitutes; the other amino groups remain free for subsequently fixing a bioactive molecule. The reaction concentration of the various linkers is between 0.02 M and 0.2 M, preferably 0.05 M.

The reaction between linker and cotton, or substitutes, is conducted for 1-3 hours, preferably 2 hours, at a temperature between ambient T °C and 70°C, preferably at 50°C, and at a pressure between atmospheric and 5 bar, preferably 3.5 bar. Reduction of the pre-oxidized groups on the cotton, or substitutes, not having reacted with the bioactive molecule or the linker is carried out by treating with reducing agents of sodium borohydride type, at a concentration of 0.02 - 0.1 M at ambient temperature and pressure for a period of 10 - 20 minutes.

The bioactive molecules, as such or in derivatized form, preferably halogenated or aminated, are reacted either directly with the cotton polymer or substitutes (the aminated form) or with the polymer-linker intermediate (halogenated form) at a concentration varying between 0.01 M and 0.5 M, preferably 0.1 M.

The reaction conditions are: time between 1 hour and 3 hours, preferably 2 hours, temperature from ambient to 70°C, preferably at 50 °C; pressure from atmospheric to 5 bar, preferably 3.5 bar.

The bioactive molecules covalently conjugated to cotton, in accordance with this procedure, are for example:

1. Antiseptics

   a - chlorhexidine gluconate, as such, for producing medical products, for example, hydrophobic gauze and bandages for external protection of wounds;
   b - benzalkonium chloride, as such, for producing sanitized garments;

2. Acaricides:

   a - benzyl benzoate, in the form of a diamine derivative [H2N-C6H5-COO-CH2-C6H5-NH2] for producing, for example, outdoor fabrics;
   b - lindane, as such (all its stereoisomers and mixtures thereof), or in its diamine derivative form [C6H6-(NH2) 2-Cl4] for the same end purpose.

3. Insecticides/insect repellents

   a - N,N-diethyl-m-toluamine in the sulfamide derivative forms

[CH3-C6H3-CON-(C2H5)2],
        |
      SO2NH2

Bromo-derivative [CH3-C6H3-CON-(C2H5)2], and amino derivative in
                                |
                               Br

different positions:

[CH3-C6H4=N-NH2-N-(C2H5)2] and [CH3-C6H3-CON-C2H5)2], for the
                                              |
                                             NH2

production of, for example, outdoor fabrics.
b. dibutyl phthalate, in the form of the amino derivative [NH2-C6H4-1,2-(CO2(CH2)3CH3)2], with the same end purpose.

The bioactive molecules covalently conjugated to viscose, in accordance with this process are, for example,

chlorhexidine, as such, as an antiseptic agent for producing antiseptic-hemostatic hydrophilic gauzes for use in direct contact with wounds.

Due to its specific characteristic of mechanical resistance, viscose lends itself to more severe oxidative treatments that provide it with hemostatic type properties (Merck Index 30th Ed, no. 7008 - Oxidized cellulose); therefore, using the same process of covalently conjugating chlorhexidine, with optimised timing of the pre-oxidation/reduction steps, a product with the twofold property (i.e. antiseptic, hemostatic) can hence be obtained and which optimises its use for direct contact with wounds.

The process is conducted in normal dyeworks equipment, consisting of temperature controlled pressure reactors equipped with loading tank, for fibre and yarn, or temperature controlled horizontal shaking baths for finished articles.

The procedure is similar to a normal dyeing process and comprises normal washes with running water at ambient temperature between the various synthesis steps.

The same process can be undertaken as circumstances require on fibre, yarn or the finished article before or after a possible dyeing or bleaching treatment.

The characteristics of the various covalent conjugates can be summarized as follows:

a. a stable bioactive molecule conjugation bond, which retains its effectiveness after numerous machine washes (even with the use of surfactants and temperatures from 60 to 90°C),

b. conservation of organoleptic and marketable properties of the fibre, yarn and fabric, in cotton and substitutes, during the treatment,

c. compatibility of the process with normal dyeworks,

d. compatibility of the process with dyeing, bleaching, sizing treatments or other procedures necessary for producing the final product: gauze, woven and non-woven fabrics etc.,

e. ability to determine concentration of covalently conjugated bioactive molecule per unit weight of fibre or yarn and of unit surface area of fabric with an immunoenzymatic method, using an antibody specific for the bioactive molecule,

f. possibility of undertaking the conjugation either on the raw product (fibre, yarn) or on a previously bleached or dyed finished product (gauze, fabric or garment),

g. not dispersing the bioactive molecules within the environment or releasing them onto skin or mucosa, hence possibility of using microbicides, acaricides and insecticides/insect repellents without risk of possible side effects.

## Examples

### 1. Derivatization of the bioactive molecules

#### a. Bromination of benzyl benzoate

21.2 g (0.2 mol) of benzyl benzoate are mixed with 2.5 g of AlCl3 in a 250 ml flask equipped with a reflux refrigerator and a dropping funnel.

96 g of bromine (0.6 mol) are added very slowly, while heating gently.

As soon as the reaction is triggered, heating is stopped. Bromine is added over a period of five hours.

The mixture is then washed three times with water before being subjected to distillation with stream.

The mixture is then dried over anhydrous CaCl2 and distilled under vacuum (p = 40 mmHg); a distillate is obtained which crystallizes in the form of a whitish-brown solid (weight = 8.3 g).

#### b. Preparation of amino-benzyl benzoate

*1. Nitration of benzyl benzoate*

32 ml of 65% HNO3 (0.33 mol) and 23 ml of 96% H2SO4 (0.23 mol) are poured into a 250 ml flask with two angled necks.

29.5 ml of benzyl benzoate (0.14 mol) are added drop-wise under vigorous stirring.

The temperature rises rapidly to 50°C and is maintained at this level for the whole period of benzyl benzoate addition.

On completion of the addition, stirring is continued for a further 30 minutes, after which the mixture is left to stand for 2 hours.

The mixture is transferred to a separating funnel; the nitro-benzyl benzoate layer is collected and washed with water containing a little Na2CO3.

This is then dried over anhydrous CaCl2 and the oil is distilled under vacuum (p = 10 mm Hg).

*2. Reduction of the nitro-derivative in amino-benzyl benzoate*

The nitro-derivative of benzyl benzoate and 10 g of granulated tin are introduced into a 250 ml flask.

About 5 g of HCl (about 4.5 ml) are added. After a few minutes the liquid boils energetically and is cooled by a water flow.

When the reaction has subsided, 4.5 ml of HCl are again added and the procedure is continued in this manner until the reaction ceases.

After heating in a water bath and when the tin is completely dissolved, 24 ml of water are added followed by a very concentrated solution of 32 g NaOH, then cooling so that the mixture does not become overheated.

About 400 ml of liquid are collected to which 80 g of NaCl are added.

The solution is brought to dryness by distillation under vacuum.

The powdered mass is extracted with methanol; the extract is filtered and brought to dryness under vacuum.

A whitish powder is obtained.

**c. Preparation of aminated lindane**

5 g of lindane (1.72 x $10^{-2}$ mol) are treated with 4.6 x $10^{-1}$ mol of 3% NH40H, i.e. 0.31 ml.

The lindane was previously dissolved in 50 ml of ethanol plus 15 ml of acetone. Under vigorous stirring, the mixture is heated in a water bath under reflux for 30 minutes, then cooled in an ice bath.

12.5 ml of 32% HCl are then gradually added under stirring.

The reaction is allowed to proceed for 15 minutes in the ice bath.

The precipitate formed is collected in a buchner funnel and recrystallized in ethanol.

Colourless crystals are obtained.

**d. Preparation of chlorosulfonyl-N,N-diethyl-m-toluamide:**

20 g of N,N-diethyl-m-toluamide (5.2 x $10^{-2}$ mol) are poured into a reactor attached to a HCl gas absorption system and equipped with a dropping funnel, a refrigerator and a mechanical stirrer.

The reactor is cooled in ice and 70 g of chlorosulfonic acid (3 x $10^{-1}$ mol) are quickly added under rapid stirring.

When the reaction slows down, the mixture is heated under reflux in a water bath for 20 minutes, after which it is allowed to cool to ambient temperature.

The mixture is poured very slowly under vigorous stirring into a mixture of 50 ml of water and 100 g of crushed ice until the excess chlorosulfonic acid reacts with the water.

The reactor is rinsed with iced water and the entire mixture is stirred again for a few minutes.

The derivative formed is extracted with $CHCl_3$ after having removed the unreacted diethyl-m-toluamide.

The extract is concentrated under vacuum (p = 20 mmHG).

A very pale yellow oil is collected.

**e. Preparation of chlorosulfonyl butyl phthalate**

14.6 g of butyl phthalate cooled in an ice bath are reacted with 70 g of chlorosulfonic acid. The acid is added fairly rapidly while maintaining the temperature between 0 and 5°C.

When the reaction begins to slow down the mixture is heated in a water bath for 20 minutes; a large quantity of HCl gas is released.

After cooling the mixture to ambient T°C, the entirety is poured into a mixture of 50 ml of water plus 100 g of ground ice, under vigorous stirring.

A brownish solid is separated off which is collected in a buchner funnel, washed with iced water and dissolved in the minimum quantity of hot ethanol.

The alcohol is distilled and a dark brown oil is obtained (w = 11.2 g).

**f. Preparation of amino butyl phthalate**

*1. Obtaining nitro-butyl phthalate*

A mixture of 15 ml NO3H (d = 1.42) and 15 ml of H2SO4 (96%) is poured into a 250 ml two necked flask equipped with reflux refrigerator and a dropping funnel, cooling to 0°C.

27.5 ml of butyl phthalate are slowly poured into the mixture of the two acids under vigorous stirring, maintaining the temperature at 0°C, in an ice bath.

When all the butyl phthalate has been added, the mixture is heated to 50°C for 20 minutes to complete the reaction.

The mixture is then poured into water.

A small whitish precipitate is separated off.

The aqueous solution is extracted with ether. The ether fraction is washed twice with water, dried over anhydrous Na2SO4 and the ether is distilled off.

A dark yellow opalescent oil remains (w = 23.9 g).

*2. Obtaining amino butyl phthalate*

5 g of NaBH4 in 50 ml of 10% NaOH are introduced into a reactor equipped with dropping funnel.

Over a period of 30 minutes, 23 g of the nitro-derivative dissolved in 50 ml of ethanol are added maintaining the temperature between 25 and 30°C, followed by a further 45 minutes of stirring.

The mixture is acidified by adding concentrated HCl to destroy excess NaBH4.

A brown oil is separated off which is collected.

The aqueous fraction is extracted twice with ether.

The two extracts are added to the oil and the mixture is concentrated by distillation under vacuum (p = 10 mm Hg).

A pale yellow solid is obtained which is recrystallized in acetone.

**2. Preparation of specific linkers**

a. preparation of melamine monohydrochloride and polymerized melamine.

*1. Preparation* of *melamine monohydrochloride.*

Dissolve 5 g of melamine in 14 ml of 8.76 N HCl with heating.

$$[5 \text{ g of melamine} = 1.19 \times 10^{-1} \text{ mol NH2}]$$

$$[14 \text{ ml of } 8.76 \text{ N HCl} = 1.22 \times 10^{-1} \text{ mol HCl}]$$

Bring the volume to 300 ml with H2O.

Dissolve the entire mixture at boiling point then leave to cool.

Collect the precipitate through a buchner funnel.

Redissolve in 300 ml of H2O and add N/1 NaOH (about 6 ml) until a small precipitate appears (about pH 4.5).

Obtained: melamine-monoHCl = 162.6 Mr

*2. Polymerization of melamine hydrochloride*

16 ml of 50% glutaraldehyde (8 x $10^{-2}$ mol) are added to the solution of melamine

HCl.

After reacting for one hour, the pH is adjusted to 8.9 - 9.0 by adding 1 M carbonate buffer at pH 9.0.

$$[\text{Ratio gluta/-NH2} = 0.66]$$

The mixture is left to react for three hours at 37°C.

The precipitate formed is recrystallized in a 1:1 dioxane:water mixture.

Obtained: polymerized melamine-HCl.

**b. preparation of tetraaminobenzene**

*1. Preparation* of *tribromoaniline*

A solution of 110 g bromine (35.3 ml) in 80 ml of CH3-COOH is prepared. 20 g of aniline and 80 ml of CH3-COOH are introduced into a 500 ml 3-necked flask.

The solution is mechanically stirred and is added drop-wise, by means of a funnel with ground glass cone, to the bromine solution while cooling the reactor with ice.

The temperature is monitored so as not to exceed 30°C.

When all the Br has been added, the reaction is left for a further 15 minutes under stirring.

The contents of the reactor are then added to 800 ml of water.

After stirring the precipitate formed is collected by filtering through a buchner funnel.

The precipitate is repeatedly washed with water, then collected once again on a buchner funnel where it is firmly squeezed to remove as much water as possible.

The still wet product is recrystallized in the minimum quantity of ethanol, then dried.

Obtained: 2,3,5-tribromoaniline - Mr 329.73.

*2. Preparation of 1, 2, 3, 5-tetraaminobenzene*

A stream of saturated ammonia gas is passed into 5 g of tribromoaniline in an EtOH solution up to saturation.

The formed tetramine derivative solution is evaporated until almost dry.

The residue is resuspended in water and collected on a buchner funnel then washed with water.

The collected solid is recrystallized in the minimum quantity of EtOH and dried at 50°C over 1205.

Obtained: 1,2,3,5-tetraaminobenzene - Mr 138

**3. Examples of conjugation of bioactive molecules to cotton or substitutes by means of linkers**

**3.1 Conjugation of bioactive molecules, derivatized to a greater or lesser extent, to cotton fibre**

*a. conjugation of the antiseptic chorhexidine to fibre rendered hydrophilic, aimed at the production* of *tampons for medical use.*

*1. Hydrophilization* of *fibre*

6 kg of cotton fibre are placed in a 60 litre dyeing reactor with continuous circulation.

The reactor is filled with 60 litres of 5N NaOH and left to react for 5 hours at 80°C.

After cooling, it is repeatedly washed until the pH of the washing waters is neutral.

*2. Conjugation of the antiseptic chlorhexidine*

Without removing the fibre from the reactor, the volume is returned to 60 litres with water and heated to 50°C, adjusting the pressure to 3.5 bar.

1.7 g per litre of sodium m-periodate are added and left to react for 10 minutes.

Three consecutive washes are carried out with water.

The volume is returned to 60 litres and 1.7 g per litre of lysine-2HCl prebuffered at pH 9.0 with sodium hydroxide are added and left to react for 2 hours again at 50°C and 3.5 bar pressure.

Two consecutive washes are carried out with water.

The volume is returned to 60 litres and 1.0 g per litre of sodium borohydride are added and left to react for 15 minutes at ambient temperature.

Two consecutive washes are carried out with water.

The volume is returned to 60 litres, and 6 ml per litre of 20% (w/v) chlorhexidine gluconate solution are added; it is left to react for one hour at 50°C and at 3.5 bar pressure.

Three washes are carried out; the fibre is unloaded from the reactor and dried.

**b. conjugation of the antiseptic benzalkonium to fibre rendered hydrophilic, aimed at the production of products for intimate hygiene.**

*1. Hydrophilization of the fibre*

60 kg of cotton fibre are placed in a 600 litre dyeing reactor with continuous circulation.

The reactor is filled with 600 litres of 5N NaOH and left to react for 12 hours at 80°C.

After cooling, the contents are repeatedly washed until washing water pH is neutral.

*2. Conjugation of the antiseptic benzalkonium*

Without removing the fibre from the reactor, the volume is returned to 600 litres with water and heated to 50°C, adjusting the pressure to 3.5 bar. 1.7 g per litre of sodium m-periodate are added and left to react for 10 minutes. The fibre is subjected to three consecutive washes with water.
The volume is returned to 600 litres with water and 1.7 g per litre of lysine-2HCl prebuffered at pH 9.0 with sodium hydroxide are added and left to react again for 2 hours at 50°C and at 3.5 bar pressure.
Two consecutive washes are carried out with water. The volume is returned to 600 litres and 1 g per litre of sodium borohydride are added and left to react for 15 minutes at ambient temperature and pressure.
Two consecutive washes are carried out with water. The volume is returned to 600 litres and 13 ml per litre of a 40% (w/v)
benzalkonium chloride solution are added; it is left to react for one hour at 50°C and at 3.5 bar pressure.
Three washes are carried out; the fibre is unloaded from the reactor and dried.

**c. conjugation of the acaricide derivatized benzyl benzoate to natural cotton fibre, aimed at the production of padding for furnishings and other similar articles**

60 kg of cotton fibre are placed in a 600 litre dyeing reactor with continuous circulation.
The reactor is filled to the rated volume with water and the temperature is brought to 50°C, adjusting the pressure to 3.5 bar.
1.7 g per litre of sodium m-periodate are added and left to react for 10 minutes. Three consecutive washes are carried out with water.
The volume is returned to its original level and 1.25 g per litre of melamine mono-HCl are added and left to react for 2 hours at 50°C at a pressure of 3.5 bar.
Two consecutive washes are carried out with water.
The volume is returned to its original level and 1.0 g per litre of sodium borohydride are added and left to react for 15 minutes at ambient temperature and pressure.
Two consecutive washes are carried out with water.
The volume is returned to 600 litres and 1.0 g per litre of Br-benzyl benzoate are added; it is left to react for one hour at 50°C and at 3.5 bar pressure.
Three washes are carried out with water; the fibre is unloaded from the reactor and dried.

**d. conjugation of the acaricide derivatized lindane to natural cotton fibre, aimed at the production of padding for furnishings and other similar articles** 60 kg of cotton fibre are placed in a 600 litre dyeing reactor with continuous circulation.

The reactor is filled to the rated volume with water and the temperature is brought to 50°C, adjusting the pressure to 3.5 bar.
1.7 g per litre of sodium m-periodate are added and left to react for 10 minutes. Three consecutive washes are carried out with water.
The volume is returned to its initial level and 1.0 g per litre of polymerized melamine mono-HCl are added and left to react for 2 hours at 50°C at a pressure of 3.5 bar.
Two consecutive washes are carried out with water.
The volume is returned to its initial level and 1.0 g per litre of sodium borohydride are added and left to react for 15 minutes at ambient temperature and pressure. Two consecutive washes are carried out with water.
The volume is returned to 600 litres and 1.0 g per litre of lindane are added; it is left to react for one hour at 50°C and at 3.5 bar pressure.
Three washes are carried out with water; the fibre is unloaded from the reactor and dried.

**e. conjugation of the insecticide/insect repellent derivatized diethyl toluamide to natural cotton fibre, aimed at the production of padding for furnishings and other similar articles**

*1st Test*

60 kg of cotton fibre is placed in a 600 litre dyeing reactor with continuous circulation.
The reactor is brought to 600 litres with water and heated to 50°C, adjusting the pressure to 3.5 bar.
1.7 g per litre of sodium m-periodate are added and left to react for 10 minutes.
The fibre is subjected to three consecutive washes with water.

The volume is returned to 600 litres with water and 1.0 g per litre of benzenetetramine are added and left to react for 2 hours at 50°C at a pressure of 3.5 bar.
Two consecutive washes are carried out with water.
The volume is returned to 600 litres and 1.0 g per litre of sodium borohydride are added and left to react for 15 minutes at ambient temperature and pressure.
Two consecutive washes are carried out with water.
The volume is returned to 600 litres and 1.0 g per litre of chlorosulfonyl-N,N-diethyl-m-toluamide are added; it is left to react for one hour at 50°C and at 3.5 bar pressure.
Three washes are carried out; the fibre is unloaded from the reactor and dried.

*2nd Test*

60 kg of cotton fibre are placed in a 600 litre dyeing reactor with continuous circulation.
The reactor is brought to 600 litres with water and heated to 50°C, adjusting the pressure to 3.5 bar.
1.7 g per litre of sodium m-periodate are added and left to react for 10 minutes.
The fibre is subjected to three consecutive washes with water.
The volume is returned to 600 litres with water and 1.0 g per litre of benzenetetramine are added and left to react for 2 hours at 50°C at a pressure of 3.5 bar.
Two consecutive washes are carried out with water.
The volume is returned to 600 litres and 1.0 g per litre of sodium borohydride are added and left to react for 15 minutes at ambient temperature and pressure.
Two consecutive washes are carried out with water.
The volume is returned to 600 litres and 1.0 g per litre of Br-N,N-diethyl-m-toluamide are added; it is left to react for one hour at 50°C at 3.5 bar pressure.
Three washes are carried out; the fibre is unloaded from the reactor and dried.

**f. conjugation of the insecticide/insect repellent derivatized dibutyl phthalate to natural cotton fibre, aimed at the production of padding for furnishings and other similar articles**

60 kg of cotton fibre are placed in a 600 litre dyeing reactor with continuous circulation.
The reactor is filled to the rated volume with water and heated to 50°C, adjusting the pressure to 3.5 bar.
1.7 g per litre of sodium m-periodate are added and left to react for 10 minutes.
The fibre is subjected to three consecutive washes with water.
The volume is returned to its initial level and 1.0 g per litre of benzenetetramine are added and left to react for 2 hours at 50°C at a pressure of 3.5 bar.
Two consecutive washes are carried out with water.
The volume is returned to its initial level and 1.0 g per litre of sodium borohydride are added and left to react for 15 minutes at ambient temperature and pressure.
Two consecutive washes are carried out with water.
The volume is returned to its initial level and 1.0 g per litre of Br-dibutyl phthalate are added; it is left to react for one hour at 50°C and at 3.5 bar pressure.
Three washes are carried out; the fibre is unloaded from the reactor and dried.

**3.2 Conjugation of the bioactive molecules, derivatized to a greater or lesser extent, to natural cotton yarn**

**a. conjugation of the antiseptic chlorhexidine to natural cotton or modal cotton yarn, aimed at the production of hydrophobic medical products (gauzes, bandages, elastic bandages) for the external protection of wounds, and medical products for other uses (laparotomy sponge, surgical instrument wrap etc)**

*1. Prebleaching the yarn*

150 kg of cotton yarn in spools are placed in a 1500 litre dyeing reactor with continuous circulation.
Water is added to the rated volume and 20 g per litre of 15% sodium hypochlorite solution are added, together with 1.5 g per litre of sodium hydroxide 36BE and 1 g per litre of Nearfil PO; it is left to react for 60 minutes at ambient temperature and pressure (first bleaching step).
The contents are then washed.
The volume is returned to its initial level and 5 g per litre of H2O2, plus 2 g per litre of sodium hydroxide 36BE and 0.5 g per litre of Nearstabil V are added; it is brought to 98°C and left to react for 30 minutes at 3.5 bar.

After leaving to cool to 80°C, the reactor is unloaded (second bleaching step).
Two consecutive washes are carried out with water.

*2. Covalent conjugation of the antiseptic chlorhexidine*

The reactor is refilled to the rated level and the contents heated to 50°C, adjusting the pressure to 3.5 bar.
1.7 g per litre of sodium m-periodate are added and left to react for 10 minutes.
Three consecutive washes are carried out with water.
The volume is returned to its initial level and 1.7 g per litre of lysine-2HCl prebuffered at pH 9.0 with sodium hydroxide are added and left to react for 2 hours at 50°C and 3.5 bar pressure.
Two consecutive washes are carried out with water.
The volume is returned to its initial level and 1.0 g per litre of sodium borohydride are added and left to react for 15 minutes at ambient temperature.
Two consecutive washes are carried out with water.
The volume is returned to its initial level and 6 ml per litre of a 20% (w/v) chlorhexidine gluconate solution are added; it is left to react for one hour at 50°C and at 3.5 bar pressure.
Three washes are carried out; the spools are unloaded from the reactor and dried. The conjugation of chlorhexidine to the polysaccharide polymer (cotton) prederivatized with lysine is obtained. In particular a conjugate having the following structure is obtained: cotton-lysine-chlorhexidine gluconate.

**b. conjugation of the antiseptic benzalkonium to natural cotton or modal cotton yarn, aimed at the production of sanitized fabrics for various types of garments (underwear, shirts, etc)**

*1. Conjugation to natural yarn (cotton* or *modal cotton) in spools.*

150 kg of natural cotton (or modal cotton) yarn in spools are placed in a 1500 litre dyeing reactor with continuous circulation.
Water is added to the rated volume and heated to 50°C adjusting the pressure to 3.5 bar.
1.7 g per litre of sodium m-periodate are added and left to react for 10 minutes.
Three consecutive washes are carried out with water.
The volume is returned to its initial level and 1.7 g per litre of lysine-2HCl prebuffered at pH 9.0 with sodium hydroxide are added and left to react for 2 hours at 50°C and at 3.5 bar pressure.
Two consecutive washes are carried out with water.
The volume is returned to its initial level, 1 g per litre of sodium borohydride are added and left to react for 15 minutes at ambient temperature and pressure.
Two consecutive washes are carried out with water.
The volume is returned to its initial level and 13 ml per litre of a 40% (w/v) benzalkonium chloride solution are added; it is left to react for one hour at 50°C and at 3.5 bar pressure.
Three washes are carried out; the spools are unloaded from the reactor and dried. The conjugation of benzalkonium chloride to the polysaccharide polymer (cotton) prederivatized with lysine is obtained. In particular a conjugate having the following structure is obtained: cotton-lysine- benzalkonium.

*2. Conjugation to yarn (cotton or modal cotton) in spools after dyeing with optical white*

150 kg of cotton (or modal cotton) yarn in spools are placed in a 1500 litre dyeing reactor with continuous circulation.
Water is added to the rated volume and 10 g per litre of sodium hypochlorite and 1 g per litre of subitol are added; it is left to react for 60 minutes at ambient temperature.
Two washes are carried out.
The volume is returned to its initial level and 1 ml per litre of concentrated sodium hydroxide, 5 g per litre of H2O2 and commercial optical white dye in a quantity of 3% (w/v) are added.
The reaction is allowed to proceed for 5 minutes under cold conditions and then brought to 98°C for thirty minutes; it is cooled to 80°C then unloaded.
Two cold washes are carried out.
The volume is returned to its initial level and 1.7 g per litre of sodium m-periodate are added and left to react for 10 minutes at 50°C and at 3.5 bar pressure.
Three cold washes are carried out.
The volume is returned to its initial level and 1.7 g per litre of lysine-2HCl prebuffered at pH 9.0 with sodium

hydroxide are added and left to react for 2 hours at 50°C and at 3.5 bar pressure.
Two cold washes are carried out.
The volume is returned to its initial level and 1.0 g per litre of sodium borohydride are added and left to react for 15 minutes at ambient temperature and pressure.
Two cold washes are carried out.
The volume is returned to its initial level and 13 ml per litre of a 40% (w/v) benzalkonium chloride solution are added which are left to react for one hour at 50°C and at 3.5 bar pressure.
Three cold washes are carried out; the spools are unloaded from the reactor and put to dry.

**c. conjugation of the acaricide derivatized benzyl benzoate to natural cotton or modal yarn, in spools, aimed at the production of fabrics for furnishings and outdoor use**

150 kg of cotton (or modal cotton) yarn in spools are placed in a 1500 litre dyeing reactor with continuous circulation.
Water is added to the rated volume, adjusting the temperature to 50°C and the pressure to 3.5 bar.
1.7 g per litre of sodium m-periodate are added and left to react for 10 minutes.
Three consecutive washes are carried out with water.
The volume is returned to its initial level and 1.27 g per litre of melamine mono HCl are added and left to react for 2 hours at 50°C at 3.5 bar.
Two consecutive washes are carried out.
The volume is returned to its initial level and 1.0 g per litre of sodium borohydride are added and left to react for 15 minutes at ambient temperature and pressure.
Two consecutive washes are carried out with cold water.
The volume is returned to its initial level and 1.0 g per litre of chlorosulfonyl-benzyl benzoate are added; it is left to react for one hour at 50°C and at 3.5 bar pressure. Three washes are carried out under cold conditions; the spools are unloaded from the reactor and dried.

**d. conjugation of the acaricide derivatized lindane to natural cotton or modal cotton yarn in spools, aimed at the production of fabrics for furnishings and outdoor use**

150 kg of cotton (or modal cotton) yarn in spools are placed in a 1500 litre dyeing reactor with continuous circulation.
Water is added to the rated volume, adjusting temperature to 50°C and pressure to 3.5 bar.
1.7 g per litre of sodium m-periodate are added and left to react for 10 minutes.
Three consecutive cold washes are carried out.
The volume is returned to its initial level and 1.0 g per litre of melamine polymerized with glutaraldehyde are added and left to react for 2 hours at 50°C at a pressure of 3.5 bar.
Two cold washes are carried out.
The volume is returned to its initial level and 1.0 g per litre of sodium borohydride are added and left to react for 15 minutes at ambient temperature and pressure.
Two consecutive cold washes are carried out.
The volume is returned to its initial level and 1.0 g per litre of lindane are added; it is left to react for one hour at 50°C and at 3.5 bar pressure.
Three cold washes are carried out; the spools are unloaded from the reactor and dried in a warm air current.

**e. conjugation of the insect repellent derivatized diethyl toluamide to natural cotton or modal cotton yarn, in spools, aimed at the production of fabrics for furnishings and outdoor use**

100 kg of cotton (or modal cotton) yarn in spools are loaded into a 1000 litre dyeing reactor with continuous circulation.
Water is added to the rated volume, with preheating to 50°C and adjusting the pressure to 3.5 bar.
1.7 g per litre of sodium m-periodate are added and left to react for 10 minutes.
Three cold washes are carried out.
The volume is returned to its initial level and 1.0 g per litre of benzenetetramine are added and left to react for 2 hours at 50°C and at a pressure of 3.5 bar.
Two cold washes are carried out.
The volume is returned to its initial level and 1.0 g per litre of sodium borohydride are added and left to react for 15 minutes at ambient temperature and pressure.

Two cold washes are carried out.
The volume is returned to its initial level and 1.0 g per litre of bromo-N,N-diethyl-m-toluamide are added; it is left to react for one hour at 50°C and at 3.5 bar pressure.
Three cold washes are carried out; the spools are unloaded from the reactor and dried in a warm air current.

**f. conjugation of the insect repellent derivatized dibutyl phthalate to natural cotton or modal cotton yarn, in spools, aimed at the production of furnishing and outdoor fabrics**

100 kg of cotton (or modal cotton) yarn in spools are loaded into a 1000 litre dyeing reactor with continuous circulation.
Water is added to the rated volume, with preheating to 50°C and adjusting pressure to 3.5 bar.
1.7 g per litre of sodium m-periodate are added and left to react for 10 minutes.
Three cold washes are carried out.
The volume is returned to its initial level and 1.0 g per litre of benzenetetramine are added and left to react for 2 hours at 50°C and at a pressure of 3.5 bar.
Two cold washes are carried out.
The volume is returned to its initial level and 1.0 g per litre of sodium borohydride are added and left to react for 15 minutes at ambient temperature and pressure.
Two cold washes are carried out.
The volume is returned to its initial level and 1.0 g per litre of chlorosulfonyl-dibutyl phthalate are added; it is left to react for one hour at 50°C and at 3.5 bar pressure. Three cold washes are carried out; the spools are unloaded from the reactor and dried in a warm air current.

**3.3 Conjugation of the bioactive molecules, derivatized to a greater or lesser extent, to hydrophilized cotton yarn**

a. conjugation of the antiseptic chlorhexidine to the hydrophilized yarn, aimed at the production of medical products (gauzes, bandages) to be used in direct contact with a wound.

*1. Hydrophilization of the yarn*

60 kg of cotton yarn in spools are loaded into a 600 litre dyeing reactor with continuous circulation.
EtOH is circulated for 30 minutes at ambient temperature and pressure.
A cold wash is carried out.
The reactor is made up to its rated volume with 5N NaOH, the temperature is brought to 80°C and left to react for 5 hours.
The contents are cold washed until the washing water is neutral.

*2. Conjugation of the antiseptic chlorhexidine*

Without removing the spools from the reactor, the volume is returned to its original level with preheating to 50°C, adjusting the pressure to 3.5 bar.
1.7 g per litre of sodium m-periodate are added and left to react for 10 minutes.
Three cold washes are carried out.
The volume is returned to its initial level and 1.7 g per litre of lysine-2HCl prebuffered at pH 9.0 with sodium hydroxide are added and left to react for 2 hours at 50°C and at 3.5 bar pressure.
Two cold washes are carried out.
The volume is returned to its initial level and 1.0 g per litre of sodium borohydride are added and left to react for 15 minutes at ambient temperature and pressure.
Two cold washes are carried out.
The volume is returned to its initial level and 6 ml per litre of a 20% (w/v) chlorhexidine gluconate solution are added which are left to react for one hour at 50°C and at 3.5 bar pressure.
Three cold washes are carried out; the spools are unloaded from the reactor and dried in a warm air current.

**3.4 Conjugation of the bioactive molecules, derivatized to a greater or lesser extent, to viscose fibre**

**a. conjugation of the antiseptic benzalkonium to viscose fibre, aimed at the production of non-wovens for personal hygiene**

60 kg of viscose fibre are placed into a 600 litre dyeing reactor with continuous circulation.
The volume is made up to its rated level, with preheating to 50°C and adjusting the pressure to 3.5 bar.
1.7 g per litre of sodium m-periodate are added and left to react under these conditions for 30 minutes.
Three cold washes are carried out.
The volume is returned to its initial level and 1.25 g per litre of melamine monohydrochloride are added and left to react for 2 hours at 50°C at a pressure of 3.5 bar.
Two cold washes are carried out.
The volume is returned to its initial level and 1.0 g per litre of sodium borohydride are added and left to react for 15 minutes at ambient temperature and pressure.
Two cold washes are carried out.
The volume is returned to its initial level and 25 ml per litre of a 40% (w/v) benzalkonium chloride solution are added which are left to react for one hour at 50°C and at 3.5 bar pressure.
Three cold washes are carried out; the fibre is unloaded from the reactor and dried in a warm air current.

*Note:*

In general, in non-wovens the viscose is present at concentrations in the order of 40/50%; this requires the conjugation of a greater quantity of antiseptic per unit weight of fibre.

b. conjugation of the acaricide benzyl benzoate to viscose fibre, aimed at the production of non-wovens for outdoor use

60 kg of viscose fibre are loaded into a 600 litre dyeing reactor with continuous circulation.
The volume is made up to its rated level, with pre-heating to 50°C and adjusting the pressure to 3.5 bar.
1.7 g per litre of sodium m-periodate are added and left to react under these conditions for 30 minutes.
Three cold washes are carried out.
The volume is returned to its initial level and 1.0 g per litre of benzenetetramine are added and left to react for 2 hours at 50°C and at a pressure of 3.5 bar.
Two cold washes are carried out.
The volume is returned to its initial level and 1.0 g per litre of sodium borohydride are added; it is left to react for 15 minutes at ambient temperature and pressure.
Two cold washes are carried out.
The volume is returned to its initial level and 1.0 g per litre of Br-benzyl-benzoate are added; it is left to react for one hour at 50°C and at 3.5 bar pressure.
Three cold washes are carried out; the fibre is unloaded from the reactor and dried in a warm air current.
Note:

In general, in non-wovens the viscose is contained in concentrations in the order of 40/50%; this requires the conjugation of a greater quantity of acaricide per unit weight of fibre.

**c. conjugation of the antiseptic chlorhexidine to the hemostatic viscose fibre aimed at the production of tampons for medical use**

60 kg of viscose fibre are loaded into a 600 litre dyeing reactor with continuous circulation.
The volume is made up to its rated level, with pre-heating to 50°C, adjusting the pressure to 3.5 bar.
3.4 g per litre of sodium m-periodate are added and left to react under said conditions for 2 hours.
Three cold washes are carried out.
The volume is returned to its initial level and 1.7 g per litre of lysine-2HCl prebuffered at pH 9.0 with sodium hydroxide are added and left to react for 2 hours at 50°C and at 3.5 bar pressure.
Two cold washes are carried out.
The volume is returned to its initial level and 0.5 g per litre of sodium borohydride are added; it is left to react for 15 minutes at ambient temperature and pressure.
Two cold washes are carried out.
The volume is returned to its initial level and 6 ml per litre of a 20% (w/v) chlorhexidine gluconate solution are

added; it is left to react for one hour at 50°C and at 3.5 bar pressure.

Three cold washes are carried out; the fibre is unloaded from the reactor and dried in a warm air current.

*Note:*

Oxidation of the cellulose polymer provides it with hemostatic properties (Merck Index, 30th Edition, 7008 - oxidized cellulose).

**3.5 Conjugation of bioactive molecules, derivatized to a greater or lesser extent, to viscose yarn**

**a. conjugation of the antiseptic chlorhexidine to the hemostatic viscose yarn, aimed at the production of gauzes for medication of wounds**

100 kg of viscose yarn in spools are loaded into a 1000 litre dyeing reactor with continuous circulation.

The volume is made up to its rated level, with preheating to 50°C and the pressure is adjusted to 3.5 bar.

1.7 g per litre of sodium m-periodate are added and left to react under said conditions for 3 hours.

Three cold washes are carried out.

The volume is returned to its initial level and 1.7 g per litre of lysine-2HCl prebuffered at pH 9.0 with sodium hydroxide are added and left to react for 2 hours at 50°C at 3.5 bar pressure.

Two cold washes are carried out.

The volume is returned to its initial level and 0.5 g per litre of sodium borohydride are added; it is left to react for 15 minutes at ambient temperature and pressure.

Two cold washes are carried out.

The volume is returned to its initial level and 6 ml per litre of a 20% (w/v) chlorhexidine gluconate solution are added and left to react for one hour at 50°C at 3.5 bar pressure.

Three cold washes are carried out; the spools are unloaded from the reactor and dried in a warm air current.

**3.6 Conjugation of the bioactive molecules, through linkers, to a finished article of cotton fabric (jersey, bath robe etc), already dyed**

**a. Conjugation of the antiseptic benzalkonium to finished articles of cotton and modal cotton dyed with optical white (underwear), aimed at the production of sanitized garments**

*1. Dyeing the article with optical white dye*

50 kg of items of underwear in pure cotton or modal cotton are loaded into a 500 litre shaking reactor for dyeing finished products.

The volume is made up to its rated level and 10 g per litre of sodium hypochlorite and 1 g per litre of subitol are added, allowing the reaction to proceed at ambient temperature for one hour.

Two cold washes are carried out.

The volume is returned to its initial volume and 1.0 g per litre of concentrated sodium hydroxide, 5 g per litre of H2O2 and 3 g per litre of commercial optical white dye are added and left to react for one hour at 90°C.

Two cold washes are carried out.

*2. Conjugation of the antiseptic benzalkonium*

The shaking bath is re-filled and the temperature brought to 50°C.

1.7 g per litre of sodium m-periodate are added and left to react for 45 minutes.

Three cold washes are carried out.

The volume is returned to its initial level and 1.7 g per litre of lysine-2HCl prebuffered at pH 9.0 with sodium hydroxide are added and left to react for 3 hours at a temperature of 50°C.

Two cold washes are carried out.

The volume is returned to its initial level and 1.0 g per litre of sodium borohydride are added and left to react for 15 minutes at ambient temperature.

Two cold washes are carried out.

The volume is returned to its initial level and 18 ml per litre of a 40% (w/v) benzalkonium chloride solution are added which are left to react for two hours at 50°C, adding antifoaming derivatives at intervals.

Three washes are carried out; the garments are unloaded from the bath and dried in a warm air current.

**b. Conjugation of the antiseptic benzalkonium to finished articles of cotton and modal cotton dyed with direct dyes (children's clothes, other types of clothing and various articles), aimed at the production of sanitized articles**

*1. Dyeing the article with direct dye*

50 kg of garments (bathrobes, leisure suits or other) are loaded into a 500 litre shaking bath for dyeing finished articles.

The volume is made up to its rated level and 1.0 g per litre of concentrated sodium hydroxide, 1 g per litre of commercial subitol and 3.0 g per litre of H2O2 are added, allowing the reaction to proceed at ambient temperature for 10 minutes and then for one hour at 80°C.

Two cold washes are carried out.

The bath is refilled and 7.0 g per litre of commercial direct dye (e.g. black) is added and the temperature brought to 80°C.

After 20 minutes of reaction time 30 g per litre of sodium chloride and after a further 10 minutes, a further 30 g per litre of the same salt are added; the reaction is left to proceed for a total of two hours.

Three cold washes are carried out.

*2. Conjugation of the antiseptic benzalkonium*

The volume is returned to its initial level and the bath is heated to 50°C.

1.7 g per litre of sodium m-periodate are added and left to react for 45 minutes.

Three cold washes are carried out.

The volume is returned to its initial level and 1.7 g per litre of lysine-2HCl prebuffered at pH 9.0 with sodium hydroxide are added and left to react for 3 hours at a temperature of 50°C.

Two cold washes are carried out.

The volume is returned to its initial level and 1.0 g per litre of sodium borohydride are added and left to react for 15 minutes at ambient temperature.

Two cold washes are carried out.

The bath is filled again and 18 ml per litre of a 40% (w/v) benzalkonium chloride solution are added; it is left to react for two hours, adding antifoaming derivatives at intervals.

Three cold washes are carried out; the garments are unloaded from the bath and dried in a warm air current.

**c. Conjugation of the antiseptic benzalkonium to finished articles of cotton and modal cotton dyed with reactive dyes (children's clothes, other types of clothing and various articles), aimed at the production of sanitized articles**

*1. Dyeing the article with reactive dye*

50 kg of garments (shirts, leisure suits, children's clothes or other) are loaded into a 500 litre shaking bath for dyeing finished products.

The volume is made up to its rated level and 1.0 g per litre of concentrated sodium hydroxide, 1.0 g of commercial subitol and 3.0 g of H2O2 are added, allowing the reaction to proceed at ambient T°C for 20 minutes and then for one hour at 80°C.

Two cold washes are carried out.

The bath is refilled and 10 g per litre of reactive dye (red or yellow) are added and the reaction under shaking conditions is begun at a temperature of 60°C.

In successive steps the following are added: 15 g per litre of sodium chloride after 10 minutes; further 15 g per litre after another 15 minutes; 5 g per litre of sodium carbonate after 30 minutes and 2 ml per litre of concentrated sodium hydroxide after 10 minutes.

The reaction is left to proceed under shaking conditions for 2 hours after which it is drained and two cold washes are carried out.

A commercial detergent in a quantity of 1 ml per litre is added and it is left under shaking conditions for 30 minutes at 80°C.

Two cold washes are undertaken.

*2. Conjugation of the antiseptic benzalkonium*

The volume is returned to its initial level and the bath is heated to 50°C. 1.7 g per litre of sodium m-periodate are added and left to react for 45 minutes while shaking.

Three cold washes are carried out.

The volume is returned to its initial level and 1.7 g per litre of lysine-2HCl prebuffered at pH 9.0 with sodium hydroxide are added and left to react for 3 hours at a temperature of 50°C.

Two cold washes are carried out.

The volume is returned to its initial level and 1.0 g per litre of sodium borohydride are added; it is left to react for 15 minutes at ambient temperature.

Two cold washes are carried out.

The bath is refilled and 18 ml per litre of a 40% (w/v) benzalkonium chloride solution are added; it is left to react for two hours while shaking, adding antifoaming derivatives at intervals.

Three washes are carried out; the garments are unloaded from the bath and dried in a warm air current.

**4. Examples of direct conjugation of bioactive molecules to cotton (without the aid of linkers)**

**a. direct conjugation of the acaricide aminated benzyl benzoate to natural yarn of cotton or modal cotton, in spools, aimed at the production of fabric for furnishings and outdoor use**

150 kg of cotton (or modal cotton) yarn in spools are loaded into a 1500 litre dyeing reactor with continuous circulation.

The volume is made up to its rated level, with preheating to 50°C and adjusting pressure to 3.5 bar. 1.7 g per litre of sodium m-periodate are added and left to react under said conditions for 10 minutes.

Three cold washes are carried out.

The volume is returned to its initial level and 1.0 g per litre of amino benzyl-benzoate are added, it is left to react for 2 hours at 50°C and 3.5 bar.

Two cold washes are carried out.

The reactor is filled again and 1.0 g per litre of sodium borohydride are added and left to react for 10 minutes at ambient temperature and pressure.

Three cold washes are carried out; the spools are unloaded from the reactor and dried in a warm air current.

**b. direct conjugation of the acaricide aminated lindane to viscose fibre, aimed at the production of non-wovens for outdoor use**

60 kg of viscose fibre are loaded into a 600 litre dyeing reactor with continuous circulation.

The volume is made up to its rated level, pre-heating to 50°C is carried out and pressure adjusted to 3.5 bar. 1.7 g per litre of sodium m-periodate are added and left to react under these conditions for 30 minutes.

Three cold washes are carried out.

The volume is returned to its initial level and 1.0 g per litre of amino lindane are added and left to react for 2 hours at 50°C and 3.5 bar.

Two cold washes are carried out.

The reactor is filled again and 1.0 g per litre of sodium borohydride are added and left to react for 10 minutes at ambient temperature and pressure.

Three cold washes are carried out; the fibre is unloaded from the reactor and dried in a warm air current.

**c. direct conjugation of the insect repellent aminated butyl phthalate to natural yarn in cotton or modal cotton, in spools, aimed at the production of fabric for furnishings and outdoor use**

100 kg of cotton (or modal cotton) yarn in spools are loaded into a 1000 litre dyeing reactor with continuous circulation.

The volume is made up to its rated level, pre-heating to 50°C is carried out and pressure adjusted to 3.5 bar.

1.7 g per litre of sodium m-periodate are added and left to react for 10 minutes.

Three cold washes are carried out.

The volume is returned to its initial level and 1.0 g per litre of amino butyl phthalate are added and left to react for 2 hours at 50°C and at 3.5 bar.

Two cold washes are carried out.

The reactor is filled again and 1.0 g per litre of sodium borohydride are added and left to react for 10 minutes

at ambient temperature and pressure.

Three cold washes are carried out; the spools are unloaded from the reactor and dried in a warm air current.

**5. Determining the conjugated bioactive molecule concentration per unit weight/surface area of cotton or substitutes, using ELISA type immunoenzymatic systems**

**5.1 Examples of preparing ELISA methods for determining the concentration of bioactive molecules**

*A) Synthesis of immunogens:*

Prepared according to the methodology known in the state of the art (e.g. D.M. Weir, Handbook of Experimental Immunology, Vol 1, Immunochemistry, Blackwell Sci. Publ. Oxford, 1978).

The following immunogens were thus prepared:

- KLH-chlorhexidine (tested immunogen) and BSA-chlorhexidine (control immunogen)
- KLH-benzalkonium and BSA-benzalkonium,
- KLH- benzyl benzoate and BSA-benzyl benzoate,
- KLH-lindane and BSA-lindane,
- KLH-diethyl toluamide and BSA-diethyl toluamide,
- KLH-dibutyl phthalate and BSA-dibutyl phthalate.
  In which: KLH = Limpet hemocyanin; BSA = bovine serum albumin.

*B). Obtaining the antibodies*

Obtained from rabbit using protocols known in the state of the art (e.g. A. Johnstone & R. Thorpe, Immunochemistry in Practice, Blackwell Sci. Publ., Oxford, 1982).

The following polyclonal antibodies from rabbit were thus obtained.

- anti-chlorhexidine,
- anti-benzalkonium,
- anti-benzyl benzoate,
- anti-lindane,
- anti-diethyl toluamide,
- anti-butyl phthalate

*C). Description of the method for determining concentration of bioactive molecules covalently conjugated to cotton and substitutes*

100 mg of the sample to be tested are weighed (fibre, yarn, or fabric) and placed at the bottom of 15 ml conical centrifuge tubes.

5 ml of a 1:5000 solution of antibodies specific for the bioactive molecule to be determined, in a phosphate buffered saline (PBS), are then added.

The tubes are placed in an incubator at 37°C for one hour.

Three washes with 5 ml of phosphate buffered saline PBS, containing 1% Tween 20, are carried out, each time removing the liquid by centrifugation at 5000 rpm for 10 minutes.

5 ml of a 1:1000 solution of rabbit antibodies conjugated to the enzyme peroxidase (HRP) (Sigma) are added and placed in an incubator at 37°C for one hour.

The liquid is removed by centrifugation at 5000 rpm for 10 minutes and three washes with PBS buffer containing Tween are carried out, each time removing the liquid by centrifugation under the same conditions.

3 ml of a solution of the enzyme substrate tetramethylbenzidine (TMB Sigma ready to use) are added and it is again incubated at 37°C for 30 minutes, shielding from the light.

The coloured liquid is transferred into spectrophotometry cuvettes and the colour intensity is read (OD) at 450 nm against water.

The colour intensity is directly proportional to the quantity of the bioactive molecule coupled to the substrate.

By constructing, in parallel, a standard determination curve for the bioactive molecules in the free state (not conjugated) their concentration when conjugated to cotton can be established.

### 5.2 Concentration of products

Table 1 shows the percentage yield of bioactive molecules bound to cotton or substitutes under the experimental conditions described, for the individual compounds produced and described in the examples.

| Conjugation by means of linkers | | |
|---|---|---|
| Product | Concentration of bioactive molecule used in the synthesis g/kg of cotton or substitute | Percentage yield of bioactive molecule bound to cotton or subs. |
| - Cotton-chlorhexidine fibre (3.1.a) | 12 | 56 % |
| - Cotton-benzalkonium fibre (3.1.b) | 52 | 45 % |
| - Cotton-benzyl-benzoate fibre (3.1.c) | 10 | 50 % |
| - Cotton-lindane fibre (3.1.d) | 10 | 46 % |
| - Cotton-diethyl toluamide fibre (3.1.e) | 10 | 53 % |
| - Cotton- dibutyl phthalate fibre (3.1.f) | 10 | 45 % |
| - Cotton-chlorhexidine yarn (3.2.a) | 12 | 44 % |
| - Cotton-benzalkonium yarn(3.2.b) (natural cotton) | 52 | 40 % |
| - Cotton-benzalkonium yarn (pre-dyed with optical white) | 52 | 42 % |
| - Cotton-benzyl benzoate yarn (3.2.c) | 10 | 48 % |
| - Cotton-lindane yarn (3.2.d) | 10 | 51 % |
| - Cotton-diethyl toluamide yarn (3.2.e) | 10 | 48 % |
| - Cotton- dibutyl phthalate yarn (3.2.f) | 10 | 50 % |
| - Cotton-chlorhexidine yarn (hydrophilized) (3.3.a) | 12 | 44 % |
| - Viscose-benzalkonium fibre (3.4.a) | 100 | 54 % |
| - Viscose-benzyl benzoate fibre (3.4.b) | 10 | 46 % |
| - Viscose-chlorhexidine fibre (3.4.c) (made hemostatic) | 12 | 52 % |
| - Viscose-chlorhexidine yarn (made hemostatic) (3.5.a) | 12 | 48 % |
| - Finished articles in cotton-benzalkonium (3.6.a) (pre-dyed with optical white) | 72 | 40 % |
| - Finished articles in cotton-benzalkonium (3.6.b) (pre-dyed with direct dye) | 72 | 44 % |
| - Finished articles in cotton-benzalkonium (3.6.c) | 72 | 46 % |

(continued)

| | | |
|---|---|---|
| (pre-dyed with reactive dye) | | |
| Direct conjugation without use of linkers | | |
| Product | Concentration of bioactive molecule used in the synthesis g/kg of cotton or substitute | Percentage yield of bioactive molecule bound to cotton or subs. |
| - cotton-benzyl benzoate yarn (4.a) | 10 | 28 % |
| - viscose-lindane fibre (4.b) | 10 | 30 % |
| - cotton-butyl phthalate yarn (4.c) | 10 | 30 % |

**6. Tests of covalent conjugation bond stability**

Determining with ELISA method the quantity of bioactive molecule covalently bound to cotton or substitutes and not released after repeated machine washes with use of detergents at a temperature of 80°C.

The various products conjugated as above (fibre, yarn, fabric, finished article) with the various bioactive molecules, either directly or bound through amino linkers were subjected to a repeated series of machine washes under the aforegiven conditions.

After every five washes, aliquots of sample were withdrawn, dried in a warm air current (about 80 to 90°C) then the quantity of bioactive molecule still bound (and not denatured) was determined by an immunoenzymtic method using antibodies specific for the bioactive molecules.

The examined products were:

*A. Products conjugated through linkers of amino structure*

- hydrophilic cotton-chlorhexidine fibre,
- hydrophilic cotton-benzalkonium fibre,
- natural cotton-benzyl benzoate fibre,
- natural cotton-lindane fibre,
- natural cotton-diethyl toluamide fibre,
- natural cotton- dibutyl phthalate fibre,
- natural cotton-chlorhexidine yarn,
- natural cotton-benzalkonium yarn,
- natural cotton-benzyl benzoate yarn,
- natural cotton-lindane yarn,
- natural cotton-diethyl toluamide yarn,
- natural cotton- dibutyl phthalate yarn,
- hydrophilized cotton-chlorhexidine yarn,
- viscose-benzalkonium fibre,
- viscose-benzyl-benzoate fibre,
- hemostatic viscose-chlorhexidine fibre,
- hemostatic viscose-chlorhexidine yarn,
- articles or clothing in optical dyed cotton-benzalkonium,
- articles or clothing in direct dyed cotton-benzalkonium
- articles or clothing in reactive dyed cotton-benzalkonium

*B. Products conjugated directly*

- natural cotton-benzyl-benzoate yarn
- viscose-lindane fibre
- natural cotton- dibutyl phthalate yarn.

In no case, after fifty machine washes, was the reduction in the conjugated bioactive molecule concentration greater than five per cent.

Between fifty and a hundred washes, the reduction in the concentration was between five and ten per cent for the various products, which is not significant.

## 7. Effectiveness tests

### 7.1 Products with antiseptic-sanitizing action

Microbicidal efficacy was assessed by determining growth inhibition of the following microorganisms: Staphylococcus aureus ATCC 25923, Staphylococcus epidermidis ATCC 12228, Escherichia coli ATCC 8739, Streptococcus pyogenes ATCC 19615, Pseudomonas aeruginosa ATCC 27853, Propionibacterium acnes ATCC 111827, Salmonella enteritidis ATCC 13076, Candida albicans ATCC 14053, Epidermophyton stockdaleae ATCC 28687, adopting the following methods: 20 $\mu$l of a fresh innoculum of the different microorganisms are deposited onto a sample of fibre or yarn or fabric conjugated with antiseptic, weighing 500 mg, and left in contact for periods of 30, 60, 90 and 120 minutes.

The infected samples, after these times, are immersed in tubes containing culture medium specific for the various microorganisms.

The media are incubated for a time and temperature suitable for the growth of the different microorganisms.

After incubation the extent of growth was determined by spectrophotometric measurement of the culture medium opalescence at OD of 620 nm.

In parallel, the growth of the following was monitored:

a. an innoculum seeded directly into culture medium (monitored growth)
b. an innoculum deposited onto 500 mg samples of fibre, yarn or fabric not conjugated with bioactive molecules (fibre/yarn/control fabric)

The products assayed were:

- hydrophilic cotton-chlorhexidine fibre,
- hydrophilic cotton-benzalkonium fibre,
- natural cotton-chlorhexidine yarn,
- natural cotton-benzalkonium yarn,
- hydrophilized cotton-chlorhexidine yarn,
- viscose-benzalkonium fibre,
- hemostatic viscose-chlorhexidine fibre,
- hemostatic viscose-chlorhexidine yarn,
- finished articles in optical dyed cotton-benzalkonium,
- finished articles in direct dyed cotton-benzalkonium
- finished articles in reactive dyed cotton-benzalkonium

The results are the following:

1. All the samples of fibre, yarn, fabric, conjugated with antiseptics, whether chlorhexidine or benzalkonium are able to totally inhibit the growth of the various microorganisms even for the contact time of thirty minutes (OD < 0.040).
2. The non-conjugated controls (fibre, yarn, fabric) all give an OD value (OD > 0.700) greater than the monitored growth (OD between 0.400 and 0.500) of the order of thirty/forty per cent, demonstrating that the normal cotton and substitutes (non derivatized) have an effect of promoting microbial growth.

### 7.2 Products with acaricidal action

Efficacy of the acaricide was assessed by determining the inhibition of larval development of Dermanyssus gallinae, Ixodes ricinus and Demodex folliculorum derived from wild autochthonous cultures.

A predetermined quantity of larvae is deposited in contact with samples of fibre or yarn or fabric conjugated with acaricides.

The samples are placed in a chamber with suitable conditions for the development of larvae, and hatching of mites is monitored.

In parallel, the same quantities of larvae are incubated in samples of normal fibre, yarn and fabric not conjugated to acaricides (controls).

The assayed products were:

| *Conjugated by means of linker bonds:* |
|---|
| cotton-benzyl benzoate fibre,<br>cotton-lindane fibre,<br>cotton benzyl benzoate yarn,<br>cotton-lindane yarn,<br>viscose-benzyl benzoate fibre, |
| *Direct conjugation without use of linkers:* |
| cotton-benzyl benzoate yarn,<br>viscose-lindane fibre |

The results show:

1. All samples of fibre, yarn, fabric, conjugated with the acaricides benzyl benzoate and lindane are able to totally inhibit the development of mite larvae placed in contact thereto.
2. The larvae deposited in contact with normal fibre, yarn or fabric (controls) have a normal life cycle.

**7.3 Products with insecticidal/insect repellent action**

The insecticidal effectiveness was assessed by determining the inhibition of larval development of Blatta orientalis, Pediculus corporis and Musca domestica derived from wild cultures.
A predetermined quantity of larvae is deposited in contact with samples of fibre or yarn or fabric conjugated to insecticides/insect repellents.
The samples are placed in a chamber with suitable conditions for the development of the larvae, and hatching of the insects is monitored.
In parallel, the same quantities of larvae are incubated in samples of fibre, yarn and fabric not conjugated to insecticides/insect repellents (controls).
The assayed products were:

| *Conjugated by means of linker bonds:* |
|---|
| - cotton-diethyl toluamide fibre<br>- cotton-dibutyl phthalate fibre<br>- cotton- diethyl toluamide yarn<br>- cotton-dibutyl phthalate yarn |
| *Direct conjugation without use of linkers::* |
| - cotton-dibutyl phthalate yarn |

The results showed:

1. All samples of fibre, yarn, fabric, conjugated with the insecticides/insect repellents diethyl toluamine and dibutyl phthalate are able to totally inhibit the development of insect larvae placed in contact thereto.
2. The larvae deposited in contact with normal fibre, yarn, or fabric (controls) had a normal life cycle.

## Claims

**1.** Industrial scale process for the covalent conjugation of bioactive substances chosen from the group consisting of microbicides, acaricides, insecticides and/or insect repellents bearing or derivatized to bear an amino or halogen group, onto supports of fibre, yarn, woven and non-woven fabric of cotton or substitutes, comprising the following steps:

- pre-treatment of the support, suitable for creating aldehyde groups on its surface,
- introduction of the bioactive substance bearing or derivatized to bear an amino group, so as to enable the pre-treated support and bioactive substance to react together,
- or, as an alternative to the preceding step, introduction of a linker bearing at least two amino groups so as to enable the pre-treated support and linker to react together,
- reduction, by introducing a reducing agent, of the bond formed between the support and bioactive substance, or, in the alternative case, between the surface and linker, as well as of any excess aldehyde groups on the support,
- optionally in the case of said alternative, introducing a bioactive substance bearing or derivatized to bear a halogen group, so as to enable the linker previously bound to the support and the bioactive substance to react together.

**2.** Process as claimed in claim 1, comprising the following steps:

a. immersing the support in 5-15 litres of water per kg of loaded support, in a temperature controllable dyeing reactor/bath,
b. partial pre-oxidation of some secondary alcohol groups present on the support, by the introduction of an oxidizing agent at a concentration of between 0.02 M and 0.2 M for a period between 5 and 120 minutes, to form aldehyde groups,
c. introducing:

c1. a linker chosen from the group consisting of lysine, melamine, melamine copolymerized with a dialdehyde in a sub-stoichiometric ratio and having a m.w of between 2000 and 10,000 Da, and tetraaminobenzene at a concentration of between 0.02 M and 0.2 M or
c2. a bioactive substance as above, bearing or derivatized to bear an amino group, at a concentration of between 0.01 M and 0.5 M,
d. reacting for a period between half an hour and 4 hours to form "Schiff base" type bonds between the partially pre-oxidized support and the substance introduced in step c1 or c2,
e. reducing either any unreacted aldehyde groups present on the support, or the "Schiff base" type bond, by the introduction of a reducing agent at a concentration of between 0.01 M and 0.5 M,
f. in the case of c1, introducing a bioactive substance as above bearing or derivatized to bear a halogen group, at a concentration of between 0.01 M and 0.5 M,
g. reacting, for a period between half an hour and 4 hours, preferably between one and three hours, to form secondary amino type bonds between the bioactive substance and the linker.

**3.** Process as claimed in claim 1 or 2 wherein the bioactive substances are chosen from the group consisting of chlorhexidene gluconate, benzalkonium chloride, benzyl benzoate, lindane, N,N,-diethyl-toluamide and dibutyl phthalate.

**4.** Process as claimed in one or more of the previous claims wherein the supports consist of materials chosen from the group consisting of cotton, modal cotton or viscose.

**5.** Process as claimed in one or more of the preceding claims wherein the pre-oxidation claimed in step a. is conducted with sodium periodate as the oxidizing agent.

**6.** Process as claimed in one or more of the preceding claims wherein the pre-oxidation claimed in step a. is conducted at a temperature between ambient temperature and 70°C

**7.** Process as claimed in claim 6 wherein the pre-oxidation is conducted at a pressure between atmospheric pressure and 5 bar, preferably at 3.5 bar.

**8.** Process as claimed in one or more of claims 4-7 wherein the support is cotton or modal cotton and in which the pre-oxidation claimed in step a. is conducted for a period of between 5 and 30 minutes.

**9.** Process as claimed in one or more of claims 4-7 wherein the support is viscose and in which the pre-oxidation claimed in step a. is conducted for a period of between 5 and 120 minutes.

**10.** Process as claimed in one or more of the previous claims wherein the reaction d. is conducted at a temperature

between ambient temperature and 70°C.

**11.** Process as claimed in claim 10 wherein the reaction is conducted at a pressure between atmospheric pressure and 5 bar, preferably at 3.5 bar.

**12.** Process as claimed in one or more of the preceding claims wherein the reduction e: is conducted with borohydride, preferably sodium borohydride.

**13.** Process as claimed in claim 12 wherein the reduction e. is conducted at a temperature between 15 and 25°C and at ambient pressure.

**14.** Process as claimed in claim 13 wherein the reduction e. takes place for a time of 10-20 minutes.

**15.** Process as claimed in one or more of the previous claims wherein after each of steps b. to e., one or more washes with water are undertaken.

**16.** Process as claimed in one or more of the previous claims wherein between steps a. and b a pre-treatment with NaOH is undertaken, in order to hydrophilize the support.

**17.** Process as claimed in one or more of claims 1-16 wherein between steps a. and b. one or more bleaching pre-treatments are undertaken.

**18.** Process as claimed in claim 17 wherein said bleaching pre-treatments comprise treatments with sodium hypochlorite and/or H2O2.

**19.** Support of fibre, yarn, woven and non-woven fabric of cotton or substitutes exhibiting microbicidal, acaricidal, insecticidal and/or insect repellent activity, said support being conjugated to bioactive substances chosen from the group consisting of microbicides, acaricides, insecticides and/or insect repellents, obtainable by the process claimed in any one of 1-18 claims.

**20.** Support as claimed in claim 19 wherein the bioactive molecule responsible for the activity is bound directly or through a diamine, triamine, tetramine or polyamine type linker.

**21.** Support as claimed in claim 20 wherein the linker is chosen from lysine, melamine, tetraaminobenzene or melamine copolymerized with dialdehyde in a sub-stoichiometric ratio and having a m.w. of between 2000 and 10,000 Da.

**22.** Support as claimed in claim 21 wherein the bioactive molecule is chosen from the group consisting of chlorhexidine gluconate, benzalkonium chloride, benzyl benzoate, lindane, N,N-diethyl-toluamide and dibutyl phthalate.

**23.** Support as claimed in claim 22 wherein the linker is lysine and the bioactive molecule is chlorhexidine gluconate, in particular the conjugate: cotton-lysisne-chlorhexidine.

**24.** Support as claimed in claim 22 wherein the linker is lysine and the bioactive molecule is benzalkonium chloride, in particular the conjugate: cotton-lysine-benzalkonium.

**25.** Support as claimed in one or more of claims 19-24 exhibiting microbicidal activity wherein the viscose support maintains intact some of the aldehyde groups created in pre-oxidation, so as to also confer to them hemostatic activity.

**26.** Products obtained by a support of fibre, yarn, woven and non-woven fabric claimed in one or more of claims 19-25.

**27.** Product as claimed in claim 26 comprising a combination of antiseptic hydrophilic viscose gauzes for medicating wounds as support of claim 25 and antiseptic hydrophobic cotton gauzes for the external protection of medication and wound in general, suitable for optimising infection prevention and the wound repair process.

## Patentansprüche

**1.** Verfahren im großtechnischen Maßstab zur kovalenten Konjugation von bioaktiven Verbindungen, welche aus der

Gruppe ausgewählt werden, welche aus Mikrobioziden, Akariziden, Insektiziden und/oder Insektenschutzmitteln, welche eine Aminogruppe oder eine Halogengruppe tragen oder so derivatisiert sind, dass diese eine Aminogruppe oder eine Halogengruppe tragen, besteht, auf Träger aus Faser, Garn, einer gewebten oder einer nicht gewebten Struktur aus Baumwolle oder Ersatzstoffen, welches die nachfolgenden Schritte umfasst:

- Vorbehandlung des Trägers, welche dazu geeignet ist, auf dessen Oberfläche Aldehydgruppen zu erzeugen,
- Zugabe der bioaktiven Verbindung, welche eine Aminogruppe träger oder so derivatisiert ist, dass diese eine Aminogruppe trägt, um es so zu ermöglichen, dass der vorbehandelte Träger und die bioaktive Verbindung miteinander reagieren,
- oder, als eine Alternative zu dem vorherigen Schritt, Zugabe eines Linkers, welcher wenigstens zwei Aminogruppen trägt, um es so zu ermöglichen, dass der vorbehandelte Träger und der Linker miteinander reagieren,
- Reduktion der Bindung, welche zwischen dem Träger und der bioaktiven Verbindung oder in dem alternativen Fall zwischen der Oberfläche und dem Linker ausgebildet ist sowie von allen überschüssigen Aldehydgruppen auf dem Träger durch die Zugabe eines reduzierenden Mittels,
- in dem Fall der Alternative optional Zugabe einer bioaktiven Verbindung, welche eine Halogengruppe trägt oder so derivatisiert ist, dass diese eine Halogengruppe trägt, um es so zu ermöglichen, dass der Linker, welcher zuvor an den Träger gebunden worden ist, und die bioaktive Verbindung miteinander reagieren.

**2.** Verfahren nach Anspruch 1, welches die nachfolgenden Schritte umfasst:

a. Eintauchen des Trägers in 5 bis 15 Liter Wasser pro kg eingeführten Träger in einem Temperatur steuerbaren Färbereaktor/-bad,
b. partielle Präoxidation einiger sekundärer Alkoholgruppen, welche auf dem Träger vorliegen, durch die Zugabe eines Oxidationsmittels in einer Konzentration zwischen 0,02 M und 0,2 M für eine Zeitspanne zwischen 5 und 120 Minuten, um Aldehydgruppen auszubilden,
c. Zugabe:

c1. eines Linkers, welcher aus der Gruppe ausgewählt wird, die aus Lysin, Melamin oder mit einem Dialdehyd in einem substöchiometrischen Verhältnis copolymerisiertem Melamin mit einem Molekulargewicht zwischen 2000 und 10.000 Da besteht, sowie von Tetraaminobenzol in einer Konzentration zwischen 0,02 M und 0,2 M oder
c2. einer bioaktiven Verbindung, wie zuvor aufgeführt, welche eine Aminogruppe trägt oder so derivatisiert ist, dass diese eine Aminogruppe trägt, in einer Konzentration zwischen 0,01 und 0,5 M,

d. Reagieren für eine Zeitspanne zwischen einer halben Stunde und 4 Stunden, um zwischen dem partiell präoxidierten Träger und der in dem Schritt c1 oder c2 eingeführten Verbindung Bindungen vom "Schiff-Basen"-Typ auszubilden,
e. Reduzieren von entweder allen unreagierten Aldehydgruppen, welche auf dem Träger vorliegen, oder der Bindung vom "Schiff-Basen"-Typ durch die Zugabe eines reduzierenden Mittels in einer Konzentration zwischen 0,01 M und 0,5 M,
f. in dem Fall von c1, Zugabe einer bioaktiven Verbindung, wie zuvor genannt, welche eine Halogengruppe trägt oder so derivatisiert worden ist, dass diese eine Halogengruppe trägt, in einer Konzentration zwischen 0,01 M und 0,5,
g. Reagieren für eine Zeitspanne zwischen einer halben Stunde und 4 Stunden, vorzugsweise zwischen einer und drei Stunden, um zwischen der bioaktiven Verbindung und dem Linker Bindungen vom sekundären Amintyp auszubilden.

**3.** Verfahren nach Anspruch 1 oder 2, wobei die bioaktiven Verbindungen aus der Gruppe ausgewählt werden, welche aus Chlorhexidingluconat, Benzalkoniumchlorid, Benzylbenzoat, Lindan, N,N-Diethyltoluolamid und Dibutylphthalat besteht.

**4.** Verfahren nach einem oder mehreren der vorhergehenden Ansprüche, wobei die Träger aus Materialien bestehen, welche aus der Gruppe ausgewählt werden, welche aus Baumwolle, modaler Baumwolle oder Viskose besteht.

**5.** Verfahren nach einem oder mehreren der vorhergehenden Ansprüche, wobei die Präoxidation, welche in dem Verfahrensschritt a. beansprucht ist, mit Natriumperiodat als Oxidationsmittel durchgeführt wird.

**6.** Verfahren nach einem oder mehreren der vorhergehenden Ansprüche, wobei die Präoxidation, welche in dem Schritt

a. beansprucht ist, bei einer Temperatur zwischen Umgebungstemperatur und 70°C durchgeführt wird.

**7.** Verfahren nach Anspruch 6, wobei die Präoxidation bei einem Druck zwischen Atmosphärendruck und 5 bar, vorzugsweise bei 3,5 bar, durchgeführt wird.

**8.** Verfahren nach einem oder mehreren der Ansprüche 4 bis 7, wobei der Träger Baumwolle oder modale Baumwolle ist, und, bei dem die Präoxidation, welche in dem Schritt a. beansprucht ist, für eine Zeitspanne zwischen 5 und 30 Minuten durchgeführt wird.

**9.** Verfahren nach einem oder mehreren der Ansprüche 4 bis 7, wobei der Träger Viskose ist, und, bei dem die Präoxidation, welche in dem Schritt a. beansprucht ist, für eine Zeitspanne zwischen 5 und 120 Minuten durchgeführt wird.

**10.** Verfahren nach einem oder mehreren der vorhergehenden Ansprüche, wobei die Reaktion d. bei einer Temperatur zwischen Umgebungstemperatur und 70°C durchgeführt wird.

**11.** Verfahren nach Anspruch 10, wobei die Reaktion bei einem Druck zwischen Atmosphärendruck und 5 bar, vorzugsweise bei 3,5 bar, durchgeführt wird.

**12.** Verfahren nach einem oder mehreren der vorhergehenden Ansprüche, wobei die Reduktion e. mit Borhydrid, vorzugsweise mit Natriumborhydrid, durchgeführt wird.

**13.** Verfahren nach Anspruch 12, wobei die Reduktion e. bei einer Temperatur zwischen 15 und 25°C und bei Umgebungsdruck durchgeführt wird.

**14.** Verfahren nach Anspruch 13, wobei die Reduktion e. für eine Zeitspanne zwischen 10 und 20 Minuten stattfindet.

**15.** Verfahren nach einem oder mehreren der vorhergehenden Ansprüche, wobei nach jedem der Schritte b. bis e. eine oder mehrere Wäschen mit Wasser vorgenommen werden.

**16.** Verfahren nach einem oder mehreren der vorhergehenden Ansprüche, wobei zwischen den Schritten a. und b. eine Vorbehandlung mit NaOH durchgeführt wird, um den Träger zu hydrophilisieren.

**17.** Verfahren nach einem oder mehreren der Ansprüche 1 bis 16, wobei zwischen den Schritten a. und b. ein oder mehrere Bleichvorbehandlungen durchgeführt werden.

**18.** Verfahren nach Anspruch 17, wobei die Bleichvorbehandlungen Behandlungen mit Natriumhypochlorit und/oder mit $H_2O_2$ umfassen.

**19.** Träger aus Faser, Garn, einer gewebten oder einer nicht gewebten Struktur aus Baumwolle oder Ersatzstoffen, welcher eine mikrobiozide, akarizide, insektizide und/oder Insektenschutz-Aktivität aufweist, wobei der Träger mit bioaktiven Verbindungen konjugiert ist, welche aus der Gruppe ausgewählt sind, welche aus Mikrobioziden, Akariziden, Insektiziden und/oder Insektenschutzmittel besteht, wobei der Träger nach einem Verfahren gemäß einem der Ansprüche 1 bis 18 erhältlich ist.

**20.** Träger nach Anspruch 19, wobei das bioaktive Molekül, welches für die Aktivität verantwortlich ist, direkt oder über einen Linker vom Diamin-, Triamin-, Tetramin- oder Polyamintyp gebunden ist.

**21.** Träger nach Anspruch 20, wobei der Linker aus Lysin, Melamin, Tetraaminobenzol oder mit Dialdehyd in einem substöchiometrischen Verhältnis copolymerisiertem Melamin mit einem Molekulargewicht zwischen 2000 und 10.000 Da ausgewählt ist.

**22.** Träger nach Anspruch 21, wobei das bioaktive Molekül aus der Gruppe ausgewählt ist, welche aus Chlorhexidingluconat, Benzalkoniumchlorid, Benzylbenzoat, Lindan, N,N-Diethyltoluolamid und Dibutylphthalat besteht.

**23.** Träger nach Anspruch 22, wobei der Linker Lysin ist und das bioaktive Molekül Chlorhexidingluconat ist, insbesondere das Konjugat: Baumwolle-Lysin-Chlorhexidin.

24. Träger nach Anspruch 22 wobei der Linker Lysin ist und das bioaktive Molekül Benzalkoniumchlorid ist, insbesondere das Konjugat: Baumwolle-Lysin-Benzalkonium.

25. Träger nach einem der Ansprüche 19 bis 24, welcher eine mikrobiozide Aktivität aufweist, wobei der Viskose-Träger einige der Aldehydgruppen, welche bei der Präoxidation erzeugt werden, intakt beibehält, so dass diesem auch hämostatische Aktivität verliehen wird.

26. Produkt erhalten durch einen Träger aus Faser, Garn, einer gewebten oder einer nicht gewebten Struktur nach einem oder mehreren der Ansprüche 19 bis 25.

27. Produkt nach Anspruch 26 enthaltend eine Mischung aus antiseptischen hydrophilen Viskose-Gazen zur medizinischen Behandlung von Wunden als Träger nach Anspruch 25 und aus antiseptischen hydrophoben Baumwolle-Gazen zum äußerlichen Schutz der Medikation und Wunde im Allgemeinen, welches zur Optimierung von Infektionsprävention und des Wundenreparaturprozesses geeignet ist.

**Revendications**

1. Procédé à l'échelle industrielle pour la conjugaison covalente de substances bioactives choisies parmi le groupe constitué de microbiocides, d'acaricides, d'insecticides et/ou d'insectifuges, portant ou dérivatisées pour porter un groupe amino ou halogène, sur des supports en fibre, en fil, en tissé et non tissé de coton ou des substituts, comportant les étapes suivantes :

   - prétraitement du support, adapté pour créer des groupes aldéhydes sur sa surface,
   - introduction de la substance bioactive portant ou dérivatisée pour porter un groupe amino, de manière à permettre au support prétraité et à la substance bioactive de réagir ensemble,
   - ou, en tant qu'alternative à l'étape précédente, introduction d'un agent de liaison portant au moins deux groupes amino de manière à permettre au support prétraité et à l'agent de liaison de réagir ensemble,
   - réduction, en introduisant un agent réducteur, de la liaison formée entre le support et la substance bioactive, ou, dans le cas alternatif, entre la surface et l'agent de liaison, ainsi que de tout groupe aldéhyde en excès sur le support,
   - facultativement dans le cas de ladite alternative, introduction d'une substance bioactive portant ou dérivatisée pour porter un groupe halogène, de manière à permettre à l'agent de liaison précédemment lié au support et à la substance bioactive de réagir ensemble.

2. Procédé selon la revendication 1, comportant les étapes suivantes consistant à :

   a. immerger le support dans 5 à 15 litres d'eau par kg de support chargé, dans un réacteur/bain de teinture pouvant être commandé en température,
   b. pré-oxyder partiellement plusieurs groupes d'alcool secondaire présents sur le support, par l'introduction d'un oxydant à une concentration comprise entre 0,02 M et 0,2 M pendant une période entre 5 et 120 minutes, afin de former des groupes aldéhydes,
   c. introduire :

      c1. un agent de liaison choisi parmi le groupe constitué de la lysine, la mélamine, la mélamine copolymérisée avec un dialdéhyde dans un rapport sub-stoechiométrique et ayant un poids moléculaire compris entre 2 000 et 10 000 Da, et de tétra-aminobenzène à une concentration comprise entre 0,02 M et 0,2 M ou
      c2. une substance bioactive telle que ci-dessus, portant ou dérivatisée pour porter un groupe amino, à une concentration comprise entre 0,01 M et 0,5 M,

   d. laisser réagir pendant une période comprise entre une demi-heure et 4 heures afin de former des liaisons de type "base de Schiff" entre le support partiellement pré-oxydé et la substance introduite à l'étape c1 ou c2,
   e. réduire soit tout groupe aldéhyde n'ayant pas réagi présent sur le support, soit la liaison de type "base de Schiff", par l'introduction d'un réducteur à une concentration comprise entre 0,01 M et 0,5 M,
   f. dans le cas de c1, introduire une substance bioactive telle que ci-dessus portant ou dérivatisée pour porter un groupe halogène, à une concentration comprise entre 0,01 M et 0,5 M,
   g. laisser réagir, pendant une période comprise entre une demi-heure et 4 heures, de préférence entre une et trois heures, afin de former des liaisons de type amine secondaire entre la substance bioactive et l'agent de

liaison.

3. Procédé selon la revendication 1 ou 2 dans lequel les substances bioactives sont choisies parmi le groupe constitué de gluconate de chlorhexidine, de chlorure de benzalconium, de benzoate de benzyle, de lindane, de N,N-diéthyltoluamide et de phtalate de dibutyle.

4. Procédé selon une ou plusieurs des revendications précédentes dans lequel les supports sont constitués de matériaux choisis parmi le groupe constitué de coton, de coton modal ou de viscose.

5. Procédé selon une ou plusieurs des revendications précédentes dans lequel la pré-oxydation revendiquée à l'étape a. est réalisée en utilisant du périodate de sodium en tant qu'oxydant.

6. Procédé selon une ou plusieurs des revendications précédentes dans lequel la pré-oxydation revendiquée à l'étape a. est réalisée à une température comprise entre la température ambiante et 70°C.

7. Procédé selon la revendication 6 dans lequel la pré-oxydation est réalisée à une pression comprise entre la pression atmosphérique et 5 bar, de préférence à 3,5 bar.

8. Procédé selon une ou plusieurs des revendications 4 à 7 dans lequel le support est du coton ou du coton modal et dans lequel la pré-oxydation revendiquée à l'étape a. est réalisée pendant une période comprise entre 5 et 30 minutes.

9. Procédé selon une ou plusieurs des revendications 4 à 7 dans lequel le support est la viscose et dans lequel la pré-oxydation revendiquée à l'étape a. est réalisée pendant une période comprise entre 5 et 120 minutes.

10. Procédé selon une ou plusieurs des revendications précédentes dans lequel la réaction d. est réalisée à une température comprise entre la température ambiante et 70 C.

11. Procédé selon la revendication 10 dans lequel la réaction est réalisée à une pression comprise entre la pression atmosphériques et 5 bar, de préférence à 3,5 bar.

12. Procédé selon une ou plusieurs des revendications précédentes dans lequel la réaction e. est réalisée à l'aide de borohydrure, de préférence du borohydrure de sodium.

13. Procédé selon la revendication 12 dans lequel la réduction e. est réalisée à une température comprise entre 15 et 25°C et à pression ambiante.

14. Procédé selon la revendication 13 dans lequel la réduction e. a lieu pendant une durée de 10 à 20 minutes.

15. Procédé selon une ou plusieurs des revendications précédentes dans lequel après chacune des étapes b. à e., un ou plusieurs lavages à l'eau sont effectués.

16. Procédé selon une ou plusieurs des revendications précédentes dans lequel entre les étapes a. et b., un prétraitement à l'aide de NaOH est effectué, afin d'hydrophiliser le support.

17. Procédé selon une ou plusieurs des revendications 1 à 16 dans lequel entre les étapes a. et b., un ou plusieurs prétraitements de blanchiment sont effectués.

18. Procédé selon la revendication 17 dans lequel lesdits prétraitements de blanchiment comportent des traitements à l'aide d'hypochlorite de sodium et/ou de $H_2O_2$.

19. Support en fibre, en fil, en tissé et non tissé de coton ou substituts présentant une activité microbiocide, acaricide, insecticide et/ou insectifuge, ledit support étant conjugué à des substances bioactives choisies parmi le groupe constitué de microbiocides, d'acaricides, d'insecticides et/ou d'insectifuges, pouvant être obtenu par le procédé revendiqué dans l'une quelconque des revendications 1 à 18.

20. Support selon la revendication 19 dans lequel la molécule bioactive responsable de l'activité est liée directement ou par l'intermédiaire d'un agent de liaison de type diamine, triamine, tétra-amine ou polyamine.

**21.** Support selon la revendication 20 dans lequel l'agent de liaison est choisi parmi la lysine, la mélamine, le tétra-aminobenzène ou la mélamine copolymérisée avec un dialdéhyde dans un rapport sub-stoechiométrique et ayant un poids moléculaire compris entre 2 000 et 10 000 Da.

**22.** Support selon la revendication 21 dans lequel la molécule bioactive est choisie parmi le groupe constitué de gluconate de chlorhexidine, de chlorure de benzalconium, de benzoate de benzyle, de lindane, de N,N-diéthyl-toluamide et de phtalate de dibutyle

**23.** Support selon la revendication 22 dans lequel l'agent de liaison est la lysine et la molécule bioactive est le gluconate de chlorhexidine, en particulier le conjugué : coton-lysine-chlorhexidine.

**24.** Support selon la revendication 22 dans lequel l'agent de liaison est la lysine et la molécule bioactive est le chlorure de benzalconium, en particulier le conjugué : coton-lysine-benzalconium.

**25.** Support selon une ou plusieurs des revendications 19 à 24 présentant une activité microbiocide, dans lequel le support de viscose maintient intacts certains des groupes aldéhydes créés lors de la pré-oxydation, de manière à également leur conférer une activité hémostatique.

**26.** Produits obtenus par un support en fibre, en fil, en tissé et non tissé revendiqué dans une ou plusieurs des revendications 19 à 25.

**27.** Produit selon la revendication 26 comportant une combinaison de toiles de viscose hydrophiles antiseptiques pour traiter des blessures en tant que support selon la revendication 25, et des toiles de coton hydrophobes antiseptiques pour la protection externe de médication et de blessure en général, adapté pour optimiser la prévention des infections et le processus de cicatrisation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4035146 A


### Non-patent literature cited in the description


- Handbook of Experimental Immunology. **D.M. Weir.** Immunochemistry. Blackwell Sci. Publ, 1978, vol. 1
- **Johnstone ; R. Thorpe.** Immunochemistry in Practice. Blackwell Sci. Publ, 1982